# EUROPÄISCHE PATENTSCHRIFT

(11) **EP 1 569 573 B1**
(45) Veröffentlichungstag und Bekanntmachung des Hinweises auf die Patenterteilung: **15.09.2010**
(21) Anmeldenummer: 03789222.1
(22) Anmeldetag: 11.12.2003
(51) Int. Cl.: A61B 19/00, A61L 2/22, A47K 7/00

(54) **VORRICHTUNG ZUM AUSBRINGEN VON DESINFEKTIONS- UND REINIGUNGSMITTELN, INSBESONDERE FÜR HÄNDE**
DEVICE FOR DISPENSING DISINFECTANTS AND CLEANSERS PARTICULARLY FOR HANDS
DISPOSITIF DISTRIBUTEUR DE PRODUITS DE DESINFECTION ET DE NETTOYAGE, EN PARTICULIER POUR LES MAINS

(30) Priorität: 13.12.2002 DE 10258272
(43) Veröffentlichungstag der Anmeldung: 07.09.2005
(73) Patentinhaber: Steripower GmbH & Co. KG, 82319 Starnberg (DE)
(72) Erfinder: Schneider, Hartmut J., 67149 Meckenheim (DE)
(74) Vertreter: Zellentin, Wiger
(86) Internationale Anmeldenummer: PCT/EP2003/014065
(87) Internationale Veröffentlichungsnummer: WO 2004/054460

(56) Entgegenhaltungen:
- US-A- 5 449 502
- US-A- 5 522 411
- US-A1- 2002 182 102
- US-B1- 6 431 189

## Beschreibung

Die vorliegende Erfindung betrifft eine Vorrichtung zum Ausbringen von Desinfektions- und Reinigungsmitteln insbesondere für Hände.

Aus der JP-A-6054772 ist ein Desinfektionsapparat für Finger bekannt, welcher aus einem Gehäuse besteht, das einen Hohlraum aufweist, in welchem Sprühdüsen zum Ausbringen von Desinfektionsmittel angeordnet sind. Bringt man die Hände in den Hohlraum, so wird von einem Sensor ein Sprühstrahl auf die Hände ausgelöst und durch Waschbewegung der Hände wird das Desinfektionsmittel auf diese verteilt. Ein weiterer derartiger Apparat ist in der US-A 5,449,502 offenbart auf der der Oberbegriff von Anspruch 1 beruht.

Solche Desinfektionsgeräte werden vornehmlich in Kliniken, aber auch bei der Lebensmittelherstellung und deren Verkauf benötigt.

Nachteilig an solchen Geräten ist zum einen, dass die zur Desinfektion verwendeten Lösemittel (Alkohole) die schützende Fettschicht der Handhaut abtragen und keine Möglichkeit der Kontrolle darüber besteht, in welchem Maße dies insbesondere bei wiederholter Desinfektion geschieht, um rechtzeitig eine Rückfettung vornehmen zu können, wobei diese Gerätschaften auch so ausgelegt sind, dass ausschließlich Desinfektionsmittel ausbringbar sind. Ein weiterer wesentlicher Nachteil besteht in der Überschussflüssigkeit. Diese gelangt an die Wandungen des Gehäusetunnels und sammelt sich am Geräteboden, von wo sie verdunstet und an die Umgebungsluft gelangt, so dass MAK-Werte nicht eingehalten werden können. Grundsätzlich wäre daher bei solchen Desinfektoren eine Luftabsaugung vorzusehen.

Die vorliegende Erfindung hat sich die Aufgabe gestellt, eine Desinfektionsvorrichtung zu schaffen, mit welcher eine Kontrolle über den oben angesprochenen Fettabtrag möglich ist, mit deren Hilfe sowohl Desinfektions-, als auch Handreinigungsmittel ausbringbar sind und die in einer Weiterentwicklung ein Absaugen der Überschussflüssigkeit oder von Gasen des Desinfektionsmittels (Alkohole) entbehrlich machen, um die Umgebungsluft vor zu hohen Konzentrationen zu schützen.

Die Lösung dieser Aufgabe gelingt mit einer Vorrichtung gemäß Hauptanspruch. Weitere vorteilhafte Ausgestaltungen finden sich in den Unteransprüchen.

Infolge dieser sehr genauen handoberflächenspezifischen Dosierung gelangt auch nur wenig Überschuss an die Wandungen, von wo aus diese kleinen Mengen zum Boden ablaufen und von dort in die Lade mit dem Absorptionsmittel, so dass auch dieses relativ selten ausgewechselt werden muss.

Infolge der Fettauswaschung durch die zum Desinfizieren verwendete alkoholische Lösung leidet die Hautoberfläche und muss rückgefettet werden.

Diese Hautbelastung durch Fettabtrag von der Oberfläche kann erfindungsgemäß berücksichtigt werden. Dazu wird vorgeschlagen, einen oder mehrere Lichtstrahlen schräg, z.B. unter einem Winkel von z. B. 45 ° C auf die Haut zu richten und derart an den Hautrillen einen Schatten zu erzeugen. Dieser Schatten ist umso stärker, je tiefer die Haut entfettet ist und stellt somit hierfür ein Maß dar. Die Erfindung schlägt daher vor, diesen Schatten mit Hilfe einer Belichtungsmessung zu erfassen, auszuwerten und entsprechend die Rückfettung über ein nach der Desinfektion auszubringendes Pflegemittel zu steuern. Gleichzeitig dient eine solche Einrichtung auch dazu, das Gerät zu sperren und die Desinfektion über die Sprühdüsen nicht auszulösen, um gesundheitliche Schäden zu vermeiden.

Um dabei die Größe derdermatologisch zu vermessenden Hand möglichst genau zu erfassen, kann im Tunnel eine Handauflage vorgesehen sein, die zum Verreiben der aufgebrachten Flüssigkeiten nach dem Vermessen zurückgeschwenkt werden kann und dazu über einen entsprechenden Mechanismus mit der Tunnelwandung z. B. seitlich und motorisch klappbar verbunden ist, oder aber der Tunnel ist mit entsprechenden Markierungen versehen, nach der die Hand ausgerichtet wird.

Die von der Düse auszubringenden Mengen an Desinfektionsmittel sind vorgeschrieben und betragen etwa 1,5 ml, was für einfache Zwecke ausreicht.

Für klinische Zwecke sind bei einfachen Eingriffen ca. 3 ml vorgesehen, für OP-Zwecke 2 mal 5 ml.

Bei den erstgenannten Zwecken ist das Überschussproblem nicht gravierend, eine permanente Geruchsbelästigung ist jedoch feststellbar.

In letzterem Falle ist jedoch ein starker Überschuss vorhanden, der auf keinen Fall zu tolerieren ist.

Bei der Verwendung von Waschflüssigkeit ist jedoch stets ein zu entsorgender Überschuss vorhanden, da die Waschmittel von Bakterien kontaminiert sind.

Ein typisches Desinfektionsmittel enthält z. B. Ethanol, Isopropanol I und II, Methylalkohol, quaternäre Ammoniumverbindungen sowie Eucerit zusammen mit etwa 30 bis 35 Gew. % H₂O. Ein für die vorliegenden Zwecke in Frage kommendes Handreinigungsmittel enthält neben Desinfektionsmitteln Tenside und ist z. B. unter der Marke "Dermotan" bei der Antiseptika, Pulheim zu beziehen. Diese Mittel sind Flüssigkeiten.

Natürlich ist es möglich, die Mittel in getrennten Vorrichtungen auszubringen, es können jedoch auch zwei Düsen in der Vorrichtung vorhanden sein, die an separate Vorratsbehälter und separate Pumpen angeschlossen sind. Im folgenden wird auf eine Vorrichtung mit nur einer Düse Bezug genommen, da die Probleme in beiden Fällen gleich sind.

Die Erfindung sieht vor, die ablaufende oder abtropfende Flüssigkeit zu sammeln und einem Behälter mit einem geeigneten Absorptionsmittel zuzuführen, wo die Stoffe gebunden werden und nicht mehr bzw. nicht mehr so stark verdunsten können. Nach einer bestimmten Anzahl von Sprühvorgängen wird das Absorbens entsorgt. Da es bei entsprechender Handhabung nicht nass ist, stellt auch das Entfernen aus der Vorrichtung kein Problem dar.

Mit besonderem Vorteil lassen sich für die vorliegenden Zwecke quellfähige Polymere auf Basis von Poly-Natriumacrylat verwenden, die als Superabsorber z. B. unter der Marke Luquasorb der BASF AG bekannt sind. Diese Superabsorber können ein Vielfaches ihres Eigengewichtes absorbieren, wobei sie zu einem unlöslichen Gel aufquellen. Ihre Verwendung in Windeln ist bekannt, es hat sich jedoch überraschenderweise herausgestellt, dass sie auch stark flüchtige Substanzen, wie z. B. die in den Desinfektionsmitteln vorhandenen Alkohole zu binden in der Lage sind. Das Absorptionsmittel kann z. B. in einer Schublade unter dem Boden des Tunnels vorgelegt werden, das abtropfende Desinfektionsmittel gelangt auf die Absorberteilchen und wird von diesen aufgenommen. Von besonderem Vorteil ist dabei, dass die Absorberteilchen trocken bleiben und von Keimen nicht kontaminiert werden können.

Die Vorrichtung kann daher eine Zählvorrichtung der ausgebrachten Flüssigkeitsmenge aufweisen, anhand derer das Erreichen der Absorptionskapazitätsgrenze des Absorbers, bei vorher festliegender Absorbermenge, feststellbar ist.

Vorteilhafterweise ist der Absorber in einem Kissen mit einem Stoffbezug enthalten, der mit dem getränkten Granulat verworfen wird.

Besonders bewährt hat sich dazu ein Spinnvliesstoff in dem der Absorber als Pulver vorliegt. Die Mengenverhältnisse sind dabei folgende: 40-60, vorzugsweise 49 Gew. % Spinnvliesstoff und 30-50, vorzugsweise 41 Gew. % Poly-Natriumacrylat.

Die Herstellung des Kissens erfolgt in einfacher Weise z. B. so, dass man dem Absorbenspulver 5-15 vorzugsweise 10 Gew. % eines Schmelzklebers (hot melt) beifügt, die Mischung auf das Vlies streut und mit einem Vliesbelag durch Erwärmen des Schmelzklebers unter gleichzeitigem Pressen bei z. B. 200°C verbindet. Der Spinnvliesstoff besteht dabei aus 55 % Viskose und 45 % Polyester.

Typischerweise werden etwa 50 g/m² Superabsorberpulver verwendet, was z. B. bei einer Kissenfläche von 19x22 cm² eine Flüssigkeitsaufnahme von 125 ml erlaubt. Durch Erhöhung der Pulvermenge können problemlos bis über 500 ml Flüssigkeitsmenge aufgefangen werden.

Wie oben gesagt, ist es vorteilhaft und für bestimmte Zwecke erforderlich, genaue Mengen an Desinfektions- oder Reinigungsmitteln auszubringen. Die Vorrichtung weist dazu eine Steuerung auf, die die Menge der Flüssigkeit erfasst, z. B. durch Zählen der Hübe einer Kolbenpumpe, um nach Erreichen der Sollmenge diese automatisch abzuschalten. Die Vorrichtung weist dazu auf ihrer Frontseite Wahlschalter oder Taster, insbesondere für klinische Zwecke berührungslos arbeitende Sensoren auf, die die Menge dem Zweck der Desinfektion oder Reinigung zuordnen.

Einmal wird dadurch die Vorschrift für die Desinfektion eingehalten, zum anderem aber auch für eine geringstmögliche Ablaufmenge an Desinfektions- oder Reinigungsmittel gesorgt.

Es wird vorgeschlagen, die Wandung des Tunnels mit Teflon oder einem den "Lotus Effekt" aufweisenden Anstrichmittel zu beschichten. Von diesem Material laufen (wasserhaltige) Flüssigkeiten besonders leicht ab und geraten somit schneller zu den Absorberteilchen.

Die Erfindung ermöglicht es, den Flüssigkeitsbedarf zum Reinigen und/oder Desinfizieren der Hände sehr genau auf die Bedürfnisse des Benutzers abzustimmen. Dies erfolgt in zweierlei Weise, wobei diese Maßnahmen gemeinsam und auch getrennt voneinander angewendet werden können.

Dabei ist im Tunnel ein Sensor eingebaut, der die Oberfläche der zu reinigenden Hände erfasst. Dies können z. B. Infrarotsensoren sein, die die (vor Inbetriebnahme der Sprühdüse (n)) von den Händen abgestrahlte Wärme erfassen und die Zeitdauer der die Düse beaufschlagenden Pumpe steuern.

Eine andere Möglichkeit besteht darin, die Hände zu beleuchten und den auf den Tunnelboden (oder die Decke) geworfenen Schatten über Lichtsensoren zu erfassen.

Insbesondere können diese Maßnahmen gemeinsam angewendet werden, um die Messfehler zu mitteln. Auf diese Weise gelingt es, die Flüssigkeitsmenge genau dem Bedarf anzupassen und somit Überdosierungen extrem klein zu halten. Insbesondere im Klinikbetrieb ist dies von großer Bedeutung, da z. B. (je nach ärztlichem Vorgehen) eine Ein- bis Dreifachdesinfektion der Hände zu erfolgen hat.

Infolge dieser sehr genauen Handoberflächenspezifischen Dosierung gelangt nur wenig Überschuss an die Wandungen, von wo aus diese kleinen Mengen zum Boden ablaufen und von dort in die Lade mit dem Absorptionsmittel, so dass auch dieses relativ selten ausgewechselt werden muss.

Die zweite Anpassung an die spezifischen Bedürfnisse des Benutzers besteht in einer optoelektronischen Erfassung der Beleuchtung der Haut des Benutzers.

Infolge der Fettauswaschung durch die zum Desinfizieren verwendete alkoholische Lösung leidet die Hauptoberfläche und muss rückgefettet werden.

Diese Hautbelastung durch Fettabtrag von der Oberfläche kann erfindungsgemäß berücksichtigt werden. Dazu wird vorgeschlagen, einen oder mehrere Lichtstrahlen schräg, z.B. unter einem Winkel von z. B. 45 ° C auf die Haut zu richten und derart an den Hautrillen einen Schatten zu erzeugen. Dieser Schatten ist umso stärker, je tiefer die Haut entfettet ist und stellt somit hierfür ein Maß dar. Die Erfindung schlägt daher vor, diesen Schatten mit Hilfe einer Belichtungsmessung zu erfassen, auszuwerten und entsprechend die Rückfettung über ein nach der Desinfektion auszubringendes Pflegemittel zu steuern. Gleichzeitig dient eine solche Einrichtung auch dazu, das Gerät zu sperren und die Desinfektion über die Sprühdüsen nicht auszulösen, um gesundheitliche Schäden zu vermeiden.

Um dabei die hinsichtlich der Erfassung ihrer Größe und der dermatologisch zu vermessenden Hand möglichst genau zu erfassen, kann im Tunnel eine Handauflage vorgesehen sein, die zum Verreiben der aufgebrachten Flüssigkeiten nach dem Vermessen zurückgeschwenkt werden kann und dazu über einen entsprechenden Mechanismus mit der Tunnelwandung z. B. seitlich und motorisch klappbar verbunden ist, oder aber der Tunnel ist mit entsprechenden Markierungen versehen, nach der die Hand ausgerichtet wird.

Da aus der Vermessung einer Hand auf die Gesamtgröße beider leicht geschlossen werden kann, genügt natürlich auch die Erfassung nur einer.

Die erfindungsgemäß geschaffene Möglichkeit, einmal die Mengen spezifisch und nach der Art und Menge auszubringen, ebenso wie die gesundheitliche Überwachung, ist von besonders großem Wert im klinischen Betrieb, ebenso wichtig aber auch in der Lebensmittelindustrie, wo eine Desinfektion im Stundenrhythmus vorgeschrieben ist. Daraus lässt sich leicht erkennen, von welcher Bedeutung einmal die Verringerung der ausgebrachten Mengen und die bequeme Entsorgung des Überschusses ebenso wie die automatisierbare individuelle Gesundheitsüberwachung ist.

Ein weiterer Aspekt der Erfindung betrifft das Gehäuse selbst für die Unterbringung der Sprühvorrichtung und das Absorptionsmittel. Aufgrund der strengen Anforderungen an die Keimfreiheit z. B. unter klinischen Bedingungen ist es erforderlich, dass das Auswechseln des das Absorptionsmittel enthaltenden Kissens nicht ohne weiteres durch beliebige Personen möglich ist.

Die Anbringung eines Schlosses verbietet sich, da dieses nicht befriedigend gereinigt werden kann. Andererseits soll das Öffnen des Gehäusedeckels leicht möglich sein und dieser am Gehäuse sicher befestigt. Eine weitere Forderung besteht darin, diesen Verschluss so zu gestalten, dass möglichst wenig Öffnungen vorhanden sind.

Die Erfindung schlägt dazu vor, das Gehäuse schalenförmig auszubilden und dieses mit einer an einer Gebäudewand anbringbaren Tragplatte zu verbinden. Dabei ist die Schale auf der einen Seite über ein Gelenk oder eine Verhakung an der Tragplatte insbesondere lösbar festgelegt und gegenüberliegend befindet sich ein in den Schalenrand eingeformter Steg mit einer kopfseitigen Nase. Die Tragplatte besitzt einen umlaufenden Kragen, der rechtwinklig nach hinten (zur Gebäudewand) absteht und die von der Gehäuseschale umgriffen wird.

Der Steg weist beidseitig Einschnitte auf und bildet zusammen mit der Nase einen Riegel. Die Nase ist dabei gegenüber der Aussenkante der Tragplatte zurückgesetzt.

Die Schale wiederum übergreift mit ihrer Seitenwandung den Rand der Tragplatte (stößt in montiertem Zustand an die Gebäudewand an) und besitzt unterhalb ihrer Aussenkante ein Fenster, in dass die Nase eingreift.

Die Aussenkante der Schale weist dazu einen oberen durchgehenden Fensterrand auf. Die Nase untergreift diesen Fensterrand und sichert so die Teile in montiertem Zustand aneinander.

Unterhalb des Fensters befindet sich eine durch Einschnitte gebildete Taste mit einer Verdickung, über welche die Nase nach innen gedrückt werden kann, um den Riegel zu öffnen.

Die Besonderheit dieses Verschlusses liegt darin, dass diese im Spritzguss herstellbar ist und äußerlich kaum erkennbar, da die Einschnitte sehr schmal sein können und das Fenster durch die Nase ausgefüllt wird.

Um die Verriegelungsfestigkeit zu steigern schlägt die Erfindung weiterhin vor, neben dem vorgenannten Steg der Tragplatte beidseitig zwei weitere anzuordnen, die ebenfalls Nasen aufweisen, die jedoch kürzer sind als diejenige des federnd ausgebildeten Mittelsteges. Diese kürzeren Nasen greifen vorteilhaft in nach außen geschlossene Mulden und sperren gegen deren obere Ränder.

Dabei sind die seitlichen Stege etwas kürzer als der mittlere, so dass auch deren Nasen geringfügig tiefer liegen.

Beim Öffnen wird über die in die Schale eingeformte Taste die Nase des mittleren Steges über die z. B. keilförmig ausgebildete Verdichtung soweit eingedrückt, dass die Schale angehoben werden kann. Man lässt dann die Nase gegen den oberen Rand des Fensters zurückfedern, wodurch dieser den Rand nach außen verformt. Erst dann können die seitlichen Nasen an der oberen Fensterkante vorbei gleiten. Diese Vorgehensweise ist von außen der Konstruktion nicht anzusehen, sie stellt damit eine Sicherung gegen unbefugtes Bedienen dar.

Unterhalb der Nasen sind Gleitkeile angeordnet, so dass beim Schließen diese die Stege nach innen drücken, um diese unter den Schalenrand einrasten zu können.

Anhand der beiliegenden Figuren wird die vorliegende Erfindung näher erläutert. Dabei zeigen als Prinzipzeichnung:
- **Figur 1**: eine erfindungsgemäße Vorrichtung in Seitenansicht und
- **Figur 2**: eine solche von vorne gesehen.
- **Figuren 3 - 5**: den Gehäuseverschluss

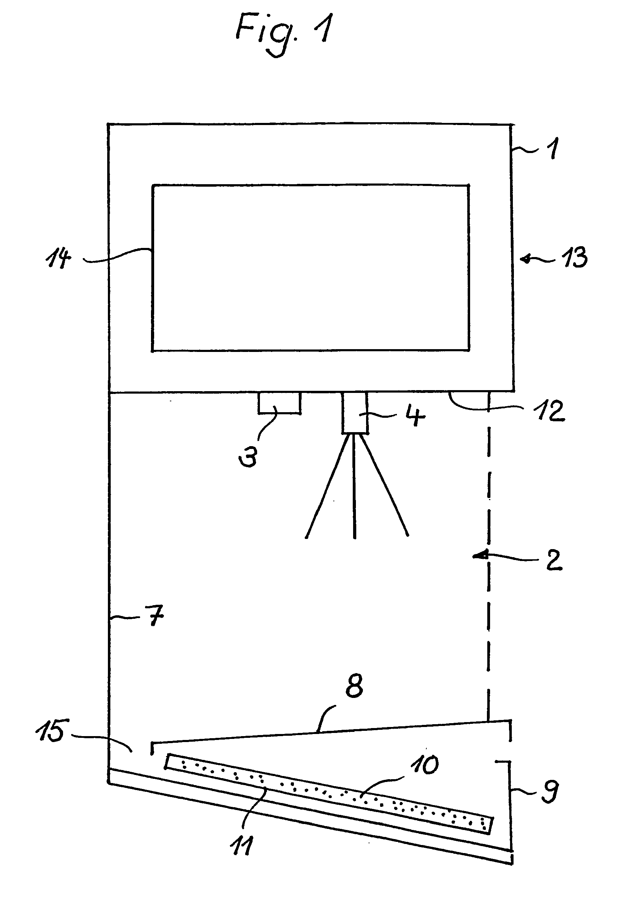

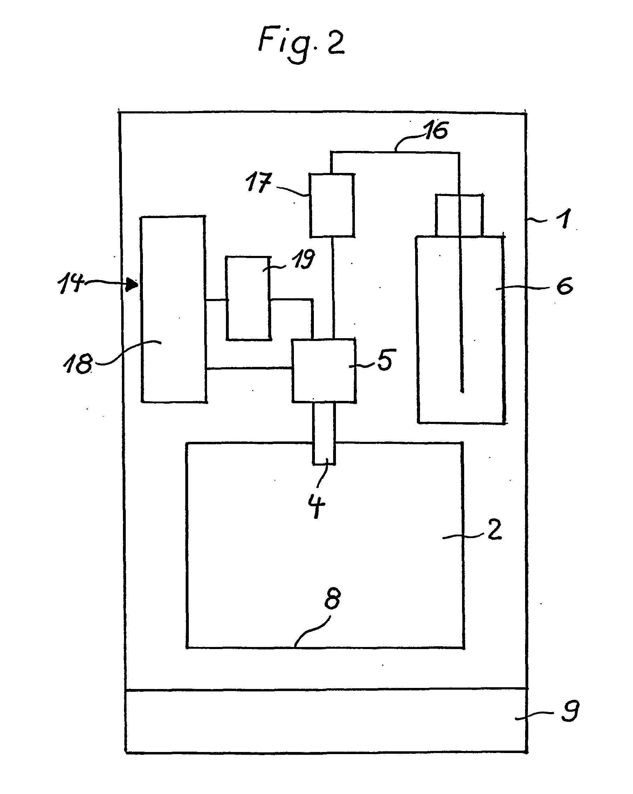

In **Figur 1** ist eine Vorrichtung mit einem Gehäuse 1 dargestellt, welches zum Teil nach außen offen ist und mit der Rückwand und dem Boden 8 und der Kopfplatte (12) sowie der gestrichelt angedeuteten Seitenwände einen Tunnel 2 bildet. Zum Oberteil des Gehäuses 1 ist eine Steuerungseinheit 14 angeordnet, die in Figur 2 näher ausgeführt ist.

An der Kopfplatte 12 befinden sich eine Sprühdüse 4 und ein Sensor 3, welcher hinter der Düse 4 angeordnet ist. Dieser Sensor 3 löst beim Annähern von Händen (prinzipiell gilt dies auch für Füße) einen oder mehrere definierte Sprühstöße aus, die auf die Hände auftreffen, um durch Waschbewegung die ausgebrachten Mittel auf der Haut zu verteilen.

Unterhalb des Sensors befindet sich ein z. B. unter einem Winkel von 10 °C zur Rückwand 7 abfallender Boden 8, der zur Rückwand als Ablauf 15 eine Öffnung freilässt. Von den Seitenwänden ablaufende Flüssigkeit gelangt auf den geneigten Boden 8 und wird zusammen mit der auf den Boden gesprühten, oder von den Händen abgetropften Flüssigkeit durch den Ablauf 15 an die darunter befindliche Lade 9 gegeben, ebenso wie auf der Rückwand 7 niedergeschlagene Flüssigkeit.

In der Lade 9 liegt das Kissen 10. Es besteht aus der Hülle 11, insbesondere aus Vliesmaterial, welches wegen seiner hohen Saugkraft aufgrund von Kapillarwirkung und hoher Reißfestigkeit bevorzugt ist. Im Inneren befindet sich das Superabsorberpulver, die obere und die untere Kissenhülle sind mit Hilfe von Schmelzkleberpartikeln verbunden.

Wie aus der Darstellung ersichtlich, kann auch die Lade 9 geneigt eingebaut sein, jedoch gegensinnig zum Boden 8, um den Fluss zu unterstützen. Die Sauggeschwindigkeit des Vlieses und des Absorbers ist jedoch so hoch und die Weitergabe von an der rückwärtigen Kante des Kissens aufgenommene Flüssigkeit, so gut, dass die Ladeneigung nur eine untergeordnete Rolle spielt.

Sämtliche mit Flüssigkeit beaufschlagten Wände, ebenso wie der Boden können vorteilhaft mit einem Polyetraflourethylenpartikel aufweisenden Anstrich verstehen sein ("Lotus Effekt") um die Haftung der Tröpfchen deutlich herabzusetzen.

Der Boden kann natürlich auch rinnenförmig gestaltet sein, um aufgesammelte Flüssigkeit möglichst schnell unter Oberflächenverringerung zu vereinigen und so die Verdunstung schon sehr früh zu verringern.

**Figur 2** zeigt das Gehäuse 1 von vorne mit seinem Tunnel 2 und der oben in diesen angeordneten Sprühdüse 4 und unter dem Boden befindlichen Lade 9. In der Steuereinheit 14 des Geräteoberteils befindet sich eine Vorratsflasche 6 oder auch mehrere davon mit gleichem oder unterschiedlichem Inhalt. Von dem Vorratsbehälter 6 führt eine Leitung 16 zu einem Rückschlagventil 17 und weiter zur Pumpe 5. Das Rückschlagventil verhindert einen unerwünschten Nachlauf.

Die Pumpe 5 wird von einer Batterie 18 gespeist, welche gleichzeitig eine Zähleinheit speist, die wiederum den Mengenftuss in der Pumpe regelt.

Eine solche Vorrichtung kann, da sie mobil ist vielseitig verwendet werden, sie braucht weder Wasserausschluss noch einen Ablauf. Die bevorzugten Einsatzgebiete sind Kliniken, wo eine zu hohe Raumluftbelastung mit den flüssigen Desinfektionsmitteln vermieden werden kann und ebenso, z. B. Lebensmittelverarbeitende Betriebe. Natürlich ist es im Sinne der Erfindung, auch vorgewaschene Hände zu reinigen/desinfizieren, wobei auch hier grundsätzlich nicht vorgetrocknet zu werden brauchen, was im Klinikumsbetrieb erforderlich ist.

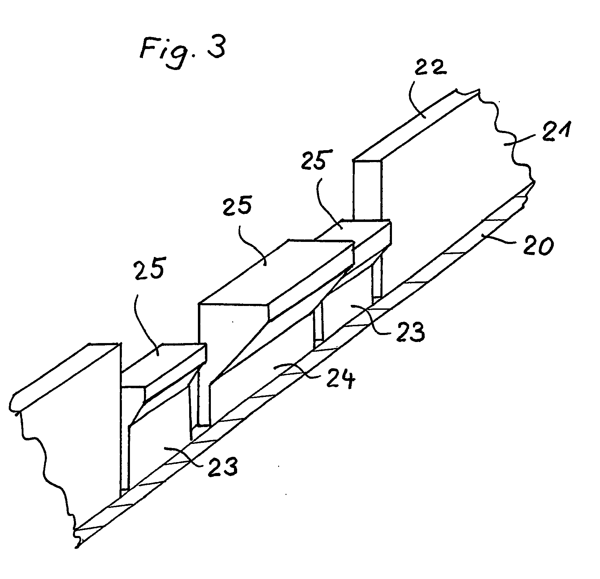

**Figur 3****,** **4 und 5** veranschaulichen den neuartigen Gehäuseverschluss. Das Gehäuse selbst besteht aus einer Tragplatte 20, die mit einer Gebäudewand verschraubt werden kann. Diese besitzt einen umlaufenden Rand 21, welcher senkrecht zur Ebene der Tragplatte 20 steht und stumpf mit ihrer Außenkante 22 gegen die Gebäudewand stößt.

Gegenüber der Außenkante 22 zurückversetzt sind drei Stege 23, 24 durch Einschnitte in den Rand 21 gebildet, die kopfseitige Nasen 25 tragen und die über Gleitkeile 29 an die Stege 23, 24 anschließen.

Die Nasen 25 sind gegenüber der Außenkante 22 zurückversetzt, wobei die mittlere Nase 25 auf einem längeren Steg 24 ruht als die benachbarten und diese somit überragt.

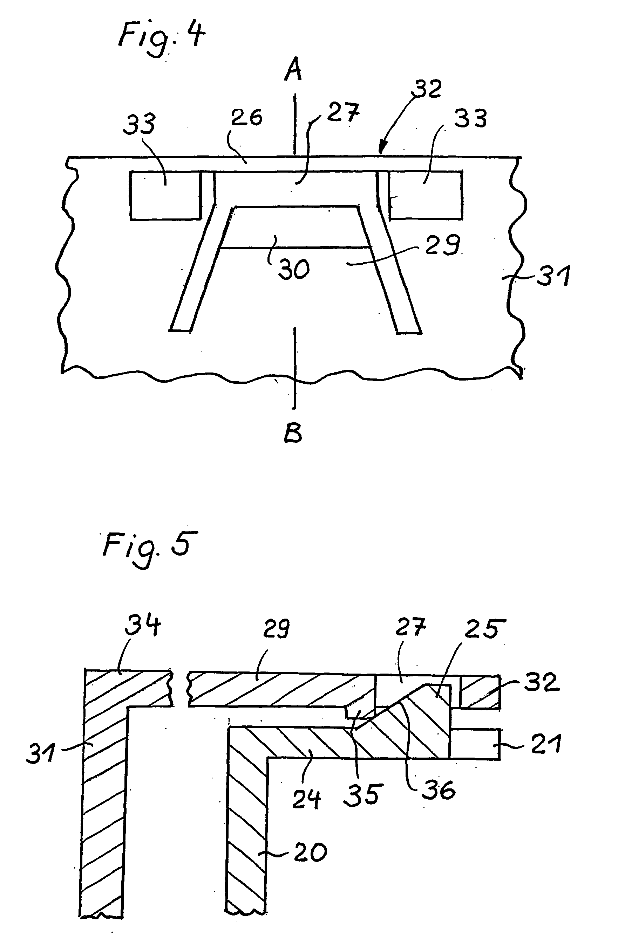

Ebenso ist aber auch die mittlere Nase 25 gegenüber der Außenkante 22 zurückversetzt und zwar mindestens um das Maß der äußeren Randleiste 26 des Fensters 2 der Gehäuseschale, die in Figur 4 angedeutet ist.

**Figur 4** zeigt dieses Fenster 27 sowie die darunter liegende Taste 29 mit ihrer Verdickung 30. Auch diese Taste 29 ist, wie die Stege 23, 24, durch einfache seitliche Einschnitte unterhalb des Fensters 27 vom Gehäuse, d.h. von der Schale 31 getrennt und federt ebenso, wie die Stege 23, 24 aufgrund der Materialeigenschaften.

Neben dem Fenster 27 befinden sich etwas tiefer, d.h. weiter vom oberen Fensterrand 32 entfernt, Mulden 33.

In geschlossenem Zustand liegt die Nase 25 des höheren Steges 24 im Fenster 27 und die Nasen 25 der kürzeren Stege 23 in den Mulden.

Die Taste 29 liegt mit ihrer Verdickung 30 gegen die mittlere Nase 25 des längeren Steges 24 an. Betätigt man die Taste 29, so drückt sie die mittlere Nase 25 aus dem Fenster 27 und der Gehäusedeckel, die Schale 31 kann soweit angehoben werden, bis die mittlere Nase in Höhe der Randleiste 26 liegt, wozu die seitlichen Mulden 33 entsprechend nach oben ein Spiel aufweisen. Danach ist die Taste 29 loszulassen, wonach die mittlere Nase gegen die Randleiste 26 des Fensterrahmens 32 drückt und diesen nach außen wölbt. Dadurch werden auch die seitlichen Nasen freigegeben und die Schale kann herausgeschwenkt werden. Drückt man die Taste 29 hingegen ständig, wie dies eigentlich für den Öffnungsvorgang zu erwarten wäre, so sperren die seitlichen Nasen der kürzeren Stege 23 gegen die Randleiste und verhinderen ein Öffnen. Diese Konstruktion stellt somit einen Sicherheitsverschluss dar und erhöht somit die Benutzungsqualität.

In **Figur 5** ist der Schnitt AB durch die Tastenanordnung wiedergegeben. Man erkennt die Schale 31 mit ihrem waagerechten Rand 34, in die die Taste 29 eingeformt ist, die unten die Verdickung 35 trägt. Daneben liegt das Fenster 27 in das die mittlere Nase 25 des Steges 24 eingreift und dieses vorzugsweise ausfüllt. Die Verdickung liegt gegen den Steg 24 oder den Gleitkeil 36 der Nase 24 an und drückt diese bei Betätigung nach unten.

Der Steg 24 (ebenso wie die daneben liegenden in Figur 3 gezeigten) ist in den rechtwinklig an die Tragplatte 20 anschließenden Rand 21 eingeformt.

Beim Schließen, d.h. dem Aufschieben der Schale 31 auf den Rand 21 der Tragplatte 20, drückt der Fensterrand 32 über den Gleitkeil 36 die Nase 25 nach unten, so dass sie in das Fenster 27 einschnappen kann.

Sämtliche Teile sind im Spritzguss aus Kunststoff hergestellt.

### Bezugszeichenlist

- 1: Gehäuse
- 2: Tunnel
- 3: Sensor
- 4: Sprühdüse
- 5: Pumpe
- 6: Vorratsbehälter
- 7: Rückwand
- 8: Boden
- 9: Lade
- 10: Kissen
- 11: Kissenhülle
- 12: Kopfplatte
- 13: Oberteil
- 14: Steuereinheit
- 15: Ablauf
- 16: Leitung
- 17: Rückschlagventil
- 18: Batterie
- 19: Zähleinheit
- 20: Tragplatte
- 21: Rand
- 22: Außenkante
- 23, 24: Stege
- 25: Nase
- 26: Randleiste
- 27: Fenster
- 29: Taste
- 30: Verdickung
- 31: Schale
- 32: Fensterrand
- 33: Mulden
- 34: Rand
- 35: Verdickung
- 36: Gleitkeil

## Patentansprüche

1. Vorrichtung zur Reinigung und/oder Desinfektion von Händen, bestehend aus einem Gehäuse (1) mit einem Tunnel (2), an dessen Wandung ein Sensor (3) und eine Sprühdüse (4) zum Ausbringen von Flüssigkeiten angeordnet sind, wobei mit dem Sensor (3) eine mit der Sprühdüse (4) verbundene Pumpe (5) auslösbar ist, die an einen Vorratsbehälter (6) angeschlossen ist, wobei
a) der Tunnel (2) einen zur Rückwand (7) abfallenden Boden (8) aufweist,
b) sich unter dem Boden (8) eine Lade (9) zum Aufsammeln von Flüssigkeit, die von der Tunnelwandung abläuft, befindet, **dadurch gekennzeichnet, dass**
c) in der Lade (9) ein Absorptionsmittel angeordnet ist, und
d) die Vorrichtung eine Beleuchtungseinrichtung aufweist, zum Beleuchten der Hand eines Benutzers, und
e) einen Belichtungsmesser umfasst, der zur Erfassung des durch die Hautrillen erzeugten Schattenwurfs dient und ein Maß für die durch mehrfaches Reinigen und/oder Desinfizieren bewirkte Fettabtragung von der Hautoberfläche bereitstellt.

2. Vorrichtung nach Anspruch 1, **dadurch gekennzeichnet, dass** diese weitere Sensoren zur Erfassung der Größe der zu reinigenden Hände aufweist.

3. Vorrichtung nach Anspruch 2, **dadurch gekennzeichnet, dass** die Sensoren die Handgröße optoelektronisch erfassen.

4. Vorrichtung nach Anspruch 1 - 3, **dadurch gekennzeichnet, dass** das Absorptionsmittel ein Polyacrylat ist.

5. Vorrichtung nach Anspruch 4, **dadurch gekennzeichnet, dass** das Polyacrylat in Granulat- oder Pulverform in einem Textilkissen (10) unter dem Boden (8) angeordnet ist.

6. Vorrichtung nach Anspruch 5, **dadurch gekennzeichnet, dass** die Kissenhülle (11) ein Spinnvliesstoff ist.

7. Vorrichtung nach Anspruch 5 oder 6, **dadurch gekennzeichnet, dass** das Kissen etwa 40-60 Gew. % Spinnvliesstoff, 30-50 Gew. % Absorptionsmittel und 5-15 Gew. % Schmelzkleber aufweist.

8. Vorrichtung nach Anspruch 7, **dadurch gekennzeichnet, dass** das Kissen 49 Gew. % Spinnvliesstoff, 41 Gew. % Polyacrylatabsorbens und 10 Gew. % Schmelzkleber aufweist

9. Vorrichtung nach Anspruch 1 - 8, **dadurch gekennzeichnet, dass** die Düse (4) mit einer mengenbegrenzenden Pumpe (5) verbunden ist.

10. Vorrichtung nach Anspruch 1 - 9 **dadurch gekennzeichnet, dass** die Tunnelwandungen mit einem Lotuseffektmittel beschichtet sind.

11. Vorrichtung nach Anspruch 1 - 10, **dadurch gekennzeichnet, dass** diese eine Zählereinrichtung zur Erfassung der ausgebrachten Flüssigkeitsmenge aufweist.

12. Vorrichtung nach Anspruch 1 - 11, **dadurch gekennzeichnet, dass** das Gehäuse (1) eine Schale (31) umfasst, die mit einer an einer Gebäudewand befestigbaren Tragplatte (20) lösbar verbunden ist,
wobei die Tragplatte (20) einen umlaufenden zu der Gebäudewand weisenden Kragen aufweist, auf den die Schale (31) aufschiebbar ist, wobei die Schale (31) den Kragen umgreift,
und wobei
die Schale (31) eine durch Einschnitte gebildete Taste (29) und ein an diese anschließendes Fenster (27) mit einem Fensterrand (32) aufweist und wobei die Tragplatte (20) in ihrem Kragen (34) einen federnden Steg (24) mit einer Nase (25) hat, wobei die Nase (25) in das Fenster (27) ragt und dabei gegen die Einschubrichtung gegen den Fensterrand (32) in sperrenden Eingriff tritt.

13. Vorrichtung nach Anspruch 12, **dadurch gekennzeichnet, dass** seitlich neben dem Steg (24) weitere Stege (23) angeordnet sind, wobei jeder Steg (23) eine Nase (25) aufweist, die gegenüber der mittleren Nase (25) des Steges (24) zurückversetzt ist;
wobei die Schale (31) eingeprägte Mulden (34) aufweist, in welche die Nasen (25) der Stege (23) eingreifen, und
wobei die Mulden (33), die Nasen (25) und der Fensterrand (32) so ausgelegt sind, daß diese erst dann aus den Mulden (33) herausbewegt werden können, wenn die Nase (25) des Steges (24) am Fensterrand (32) durch Loslassen der Taste (29) anliegt und den Fensterrand (32) nach außen verformt.

## Claims

1. Device for cleaning and/or disinfecting hands, consisting of a housing (1) having a tunnel (2) on the wall of which are arranged a sensor (3) and a spray nozzle (4) for dispensing liquids, wherein the sensor (3) can be used to trigger a pump (5) that is connected to the spray nozzle (4) and attached to a reservoir container (6), whereby
a) the tunnel (2) has a floor (8) sloping in the direction of the back wall (7);
b) a drawer (9) for collecting liquid that runs off the tunnel wall is situated underneath the floor (8), **characterized in that**
c) an absorption agent is arranged in the drawer (9);
d) the device has an illumination device for illuminating the hand of a user; and
e) comprises an exposure meter that serves for detecting the shadow produced by the skin grooves and provides a measure for the abrasion of lipid from the skin surface that is effected by multiple cleaning and/or disinfecting.

2. Device according to claim 1, **characterized in that** it has further sensors for detecting the size of the hands to be cleaned.

3. Device according to claim 2, **characterized in that** the sensors detect the hand size by optoelectronic means.

4. Device according to claim 1-3, **characterized in that** the absorption agent is a polyacrylate.

5. Device according to claim 4, **characterized in that** the polyacrylate is arranged in the form of a granulate or powder in a textile sachet (10) underneath the floor (8).

6. Device according to claim 5, **characterized in that** the envelope (11) of the sachet is a spunbonded material.

7. Device according to claim 5 or 6, **characterized in that** the sachet has approximately 40-60 % by weight spunbonded material, 30-50 % by weight absorption agent, and 5-15 % by weight hot-melt-type adhesive.

8. Device according to claim 7, **characterized in that** the sachet has approximately 49 % by weight spunbonded material, 41 % by weight polyacrylate absorbent, and 10 % by weight hot-melt-type adhesive.

9. Device according to claim 1-8, **characterized in that** the nozzle (4) is connected to a restricting pump (5).

10. Device according to claim 1-9, **characterized in that** the tunnel walls are coated with a Lotus effect agent.

11. Device according to claim 1-10, **characterized in that** it has a counting device for detecting the dispensed amount of liquid.

12. Device according to claim 1-11, **characterized in that** the housing (1) comprises a cup (31) that is connected in a detachable manner to a support plate (20) that can be secured to a wall of a building,
wherein the support plate (20) has a circumferential collar that faces the wall of the building and onto which the cup (31) can be pushed, whereby the cup (31) encompasses the collar,
and wherein
the cup (31) has a key (29) that is formed by incisions and, adjacent thereto, a window (27) with a window edge (32),
and wherein the support plate (20) has an elastic fin (24) with a lug (25) in its collar (34), whereby the lug (25) projects into the window (27) and thus engages against the window edge (32) against the insertion direction in a locking engagement.

13. Device according to claim 12, **characterized in that**, to the side of the fin (24), further fins (23) are arranged, wherein each fin (23) has a lug (25) that is situated back at an offset with respect to the middle lug (25) of the fin (24);
wherein the cup (31) has embossed depressions (34) that are engaged by the lugs (25) of the fins (23), and
wherein the depressions (33), the lugs (25), and the window edge (32) are designed such that these cannot be moved out of the depressions (33) until the lug (25) of the fin (24) touches against the window edge (32) by letting go of the key (29) and deforms the window edge (32) in outward direction.

## Revendications

1. Dispositif pour le nettoyage et/ou la désinfection des mains, constituée d'un boîtier (1) comprenant un tunnel (2) contre la paroi duquel est disposé un capteur (3) et une buse de pulvérisation (4) pour l'expulsion de liquides, une pompe reliée à la buse de pulvérisation (4) pouvant être déclenchée avec le capteur (3), la pompe étant raccordée à un récipient de réserve (6),
a) le tunnel (2) présentant une base (8) qui descend en direction de la paroi dorsale (7) ;
b) un tiroir (9) pour la récupération du liquide qui s'écoule à partir de la paroi du tunnel se trouvant en dessous de la base (8),
**caractérisé en ce que**
c) un agent d'absorption est disposé dans le tiroir (9), et
d) le dispositif présente un mécanisme d'éclairage pour l'éclairage de la main d'un utilisateur et
e) comprend un dispositif de mesure de l'éclairage qui sert à enregistrer la zone d'ombre générée par les lignes de la main et qui procure une mesure pour l'élimination des graisses de la surface de la peau, que l'on obtient grâce à plusieurs nettoyages et/ou désinfections.

2. Dispositif selon la revendication 1, **caractérisé en ce qu'**il présente des capteurs supplémentaires pour enregistrer la grandeur des mains à nettoyer.

3. Dispositif selon la revendication 2, **caractérisé en ce que** les capteurs enregistrent la grandeur de la main par voie optoélectronique.

4. Dispositif selon les revendications 1 à 3, **caractérisé en ce que** l'agent d'absorption est un polyacrylate.

5. Dispositif selon la revendication 4, **caractérisé en ce que** le polyacrylate est disposé, sous la forme d'un produit de granulation ou sous forme pulvérulente, dans un coussin en textile (10) en dessous de la base (8).

6. Dispositif selon la revendication 5, **caractérisé en ce que** l'enveloppe du coussin (11) est un non-tissé.

7. Dispositif selon la revendication 5 ou 6, **caractérisé en ce que** le coussin présente un non-tissé à concurrence d'environ 40 à 60 % en poids, un agent d'absorption à concurrence de 30 à 50 % en poids et une colle thermofusible à concurrence de 5 à 15 % en poids.

8. Dispositif selon la revendication 7, **caractérisé en ce que** le coussin présente un non-tissé à concurrence de 49 % en poids, un absorbant à base de polyacrylate à concurrence de 41 % en poids et une colle thermofusible à concurrence de 10 % en poids.

9. Dispositif selon les revendications 1 à 8, **caractérisé en ce que** la buse (4) est reliée à une pompe (5) délimitant la quantité.

10. Dispositif selon les revendications 1 à 9, **caractérisé en ce que** les parois du tunnel sont enduites d'un agent à effet lotus.

11. Dispositif selon les revendications 1 à 10, **caractérisé en ce que** le dispositif en question présente un dispositif de comptage pour l'enregistrement de la quantité de liquide appliquée.

12. Dispositif selon les revendications 1 à 11, **caractérisé en ce que** le boîtier (1) présente une coquille (31) qui est reliée de manière amovible à une plaque de support (20) qui peut être fixée à une paroi du boîtier,
dans lequel la plaque de support (20) présente un rebord périphérique orienté en direction de la paroi du boîtier, sur lequel on peut faire glisser la coquille (31), la coquille (31) venant enserrer le rebord,
et dans lequel
la coquille (31) présente une touche (29) formée par des entailles et une fenêtre (27) qui s'y raccorde et qui comprend un encadrement de fenêtre (29),
et dans lequel la plaque de support (20) possède, dans son collet (34), une nervure élastique (24) comportant un nez (25), le nez (25) faisant saillie dans la fenêtre (27) et entrant en l'occurrence en engrènement de verrouillage à l'encontre de la direction d'insertion avec l'encadrement de la fenêtre.

13. Dispositif selon la revendication 12, **caractérisé en ce que** des nervures supplémentaires (23) sont disposées latéralement à côté de la nervure (24), chaque nervure (23) présentant un nez (25) qui est décalé vers l'arrière par rapport au nez médian (25) de la nervure (24) ;
dans lequel la coquille (31) présente des renfoncements imprimés (34) dans lesquels viennent s'insérer les nez (25) des nervures (23), et
dans lequel les renfoncements (33), les nez (25) et l'encadrement de fenêtre (32) sont dimensionnés de telle sorte qu'ils ne peuvent sortir des renfoncements (33) que lorsque le nez (25) de la nervure (24) vient s'appuyer contre l'encadrement de fenêtre (32) en relâchant la touche (29) et lorsque le l'encadrement de fenêtre (32) subit une déformation vers l'extérieur.
